(19) Europäisches Patentamt
European Patent Office
Office européen des brevets

(11) EP 4 509 595 A1

(12) EUROPEAN PATENT APPLICATION
published in accordance with Art. 153(4) EPC

(43) Date of publication:
19.02.2025 Bulletin 2025/08

(51) International Patent Classification (IPC):
$C12N\ 5/00\ ^{(2006.01)}$    $C12N\ 5/078\ ^{(2010.01)}$

(21) Application number: 23788588.4

(52) Cooperative Patent Classification (CPC):
C12N 5/00; C12N 5/06

(22) Date of filing: 12.04.2023

(86) International application number:
PCT/KR2023/004918

(87) International publication number:
WO 2023/200239 (19.10.2023 Gazette 2023/42)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 12.04.2022 KR 20220045251

(71) Applicant: Artblood Inc.
Seoul 06745 (KR)

(72) Inventors:
• BAEK, Eun Jung
  Seoul 06717 (KR)
• KIM, Suyeon
  Seoul 05545 (KR)

(74) Representative: ZBM Patents - Zea, Barlocci &
Markvardsen
Rambla de Catalunya, 123
08008 Barcelona (ES)

Remarks:
The complete document including Reference
Table(s) and the Sequence Listing(s) can be
downloaded from the EPO website

(54) NUCLEATED CELL DEATH INDUCTION METHOD USING LOW TEMPERATURE TREATMENT

(57)    The present invention relates to a nucleated cell death induction method in which a low temperature treatment is performed on incubating blood cells such that only nucleated cells are damaged and enucleated blood cells are not damaged. According to the present invention, nucleated cell death can be induced by means of a simple, cost-effective and safe method.

FIG. 4A

## Description

### TECHNICAL FIELD

The present disclosure relates to a method for killing only nucleated cells and collecting only enucleated blood cells by transferring incubating blood cells to a low temperature.

### BACKGROUND ART

Blood transfusion is a very important treatment for patients who are lacking blood for various reasons. However, blood donated from blood donors has problems such as a shortage of blood supply due to a decrease in the number of blood donations and aging population, the risk of transmission of infectious diseases through blood transfusion, and other side effects of blood transfusion. For this reason, research is being conducted to mass-produce blood cells such as erythrocytes and platelets from stem cells in vitro.

In particular, in order to mass-produce erythrocytes or platelets, there may be selected a method of differentiating immortal cell lines formed by overexpressing oncogenes in stem cells or erythroid progenitor cells, or induced pluripotent stem cells formed by overexpressing cancer genes. In theory, the erythrocytes produced have no nucleus through an enucleation process, and accordingly, if only the erythrocytes are filtered well and transfused, clinical risks such as causing cancer will not be caused. However, nucleated cells may remain in the final product and a simple, cost-effective and safe method is needed to remove the nucleated cells as much as possible.

A leukocyte-reducing filter, and the like may be used to collect only the enucleated erythrocytes or platelets. However, this filter does not have 100% of a removal rate of leukocytes, and if there are relatively many nucleated cells among cells before filtering, the filter efficiency decreases and the probability that nucleated cells will not be filtered out in the product after filtering increases.

In addition, it has been reported in many literatures that blood for transfusion produced in vitro through manipulation such as overexpression of oncogenes may be used safely by killing nucleated cells through irradiation before transfusion, which has not been verified.

Cell proliferation and survival are affected by various factors, and among the factors, a temperature is an environmental factor with the greatest influence. Enucleated blood cells require relatively fewer metabolites than nucleated cells, which are relatively stable when stored in a refrigerator. Therefore, the present inventors completed the present disclosure by selecting a low temperature treatment method to damage only nucleated cells in a final blood product.

### DISCLOSURE OF THE INVENTION

#### TECHNICAL GOALS

An aspect to be achieved by the present disclosure is to provide a nucleated cell death induction method so as to damage only nucleated cells and not to damage enucleated blood cells in incubating blood cells.

#### TECHNICAL SOLUTIONS

In order to solve the aspect, the present inventors propose a method for killing only nucleated cells by a low temperature treatment of incubating blood cells.

Accordingly, the present disclosure provides a method for inducing the death of nucleated cells in enucleated blood cells

including incubating hematopoietic stem cells or progenitor cells into enucleated blood cells; and
killing nucleated cells by a low temperature treatment of the incubating blood cells.

#### EFFECTS OF THE INVENTION

According to the present disclosure, it is possible to provide a method for inducing the death of nucleated cells by means of a simple, cost-effective and safe method.

### BRIEF DESCRIPTION OF DRAWINGS

FIG. 1 shows results of confirming that HPV16 E6/E7 is expressed even after Tet off by removing doxycycline.

FIG. 2 shows results of examining the degree of hemolysis of erythrocytes after irradiation.

FIG. 3 shows results of a study on apoptosis according to a radiation dose of nucleated cells.

FIG. 4 shows results of a study on apoptosis according to a low temperature treatment (4°C) of nucleated cells.

FIG. 5 shows results of a study on apoptosis according to irradiation and a low temperature treatment.

FIG. 6 shows staining images of erythroid progenitor cells passing through a leukocyte-reducing filter and results of a study of confirming the presence or absence of nucleated cells after passing through a leukocyte-reducing filter after a low temperature treatment (4°C).

## MODE FOR CARRYING OUT THE INVENTION

Hereinafter, the present disclosure will be described in detail.

The present disclosure provides a method for inducing the death of nucleated cells in enucleated blood cells,

including incubating hematopoietic stem cells or progenitor cells into enucleated blood cells; and

killing nucleated cells by a low temperature treatment of the incubating blood cells.

In the present disclosure, the "blood cells" refer to platelets, erythrocytes and progenitor cells.

In the present disclosure, the "hematopoietic stem cells (HSCs)" refer to cells that may potentially differentiate into major components of blood.

In an embodiment of the present disclosure, the hematopoietic stem cells or progenitor cells may be isolated from bone marrow, umbilical cord blood, or peripheral blood. In an embodiment of the present disclosure, the hematopoietic stem cells or progenitor cells may be derived from all cell source-embryonic stem cells capable of differentiating into erythrocytes or platelets, adult stem cells, erythroid progenitor cell lines, platelet progenitor cell lines, induced pluripotent stem cells (iPS cells), or the like. For example, in the present disclosure, the hematopoietic stem cells may be isolated from bone marrow, umbilical cord blood, or peripheral blood, or derived from adult stem cells, embryonic stem cells, or iPS cells. In addition, for example, in the present disclosure, the progenitor cells may be isolated from bone marrow, umbilical cord blood or peripheral blood, or derived from adult stem cells, erythroid progenitor cell lines, platelet progenitor cell lines, embryonic stem cells or iPS cells.

In the present disclosure, the "progenitor cell" is an undifferentiated cell with self-replication and differentiation, but is an ultimately differentiated cell of which a type of finally differentiated cell has already been determined.

The erythrocytes are formed from hematopoietic stem cells through several differentiation steps, but the nucleus gradually condenses and the cell size decreases through burst forming unit erythroid (BFU-E), colony forming unit erythroid (CFU-E), proerythroblast, basophilic erythroblast, polychromatic erythroblast, and orthochromatic erythroblast. The condensed nucleus is enucleated and then becomes a reticulocyte, and when the remaining RNA and micro-organelles disappear, the reticulocyte has a biconcave shape to become a mature erythrocyte (red blood cell, RBC).

In the present disclosure, the progenitor cells may be blood cells before enucleation. In addition, the progenitor cells include erythroid progenitor cells or platelet progenitor cells. The erythroid progenitor cells may be burst forming unit erythroids (BFU-E), colony forming unit erythroids (CFU-E), proerythroblasts, basophilic erythroblasts, polychromatic erythroblasts, orthochromatic erythroblasts or mixtures thereof.

Therefore, in an embodiment of the present disclosure, the erythroid progenitor cells may be selected from the group consisting of burst forming unit erythroids (BFU-E), colony forming unit erythroids (CFU-E), proerythroblasts, basophilic erythroblasts, polychromatic erythroblasts, and orthochromatic erythroblasts.

In another embodiment of the present disclosure, the enucleated blood cells include erythrocytes or platelets. The enucleated erythrocytes may be selected from the group consisting of reticulocytes and erythrocytes.

The hematopoietic stem cells maintain self-renewal within the body and produce erythrocytes and other blood cells throughout life, but may not form a stem cell niches in the body in vitro, and thus do not maintain self-renewal and differentiate to produce a limited number of blood cells. Accordingly, an immortalized erythroid progenitor cell line may be used to induce hematopoietic stem cells to differentiate into blood cells, but increase the production of blood cells while extending the cell proliferation period through regulation of genes related to cell proliferation. However, in this case, since overexpression of oncogenes was used, there is a problem in safety if the final product may not be transfused by completely removing the nucleus.

Accordingly, an embodiment of the present disclosure includes killing nucleated cells by a low temperature treatment of the incubating blood cells at 0°C or higher and 15°C or lower. The incubating blood cells may be understood as a concept including both enucleated cells and non-enucleated cells in a continuous process of incubating hematopoietic stem cells or progenitor cells into enucleated blood cells.

In an embodiment of the present disclosure, the nucleated cells may be erythroid progenitor cells or platelet progenitor cells that overexpress HPV16 E6/E7. HPV16 E6/E7 refers to a HPV virus gene E6/E7, which is a cancer-causing gene that is not expressed in humans. Desirably, the overexpressed gene required for constructing an erythroid progenitor cell line may be HPV16 E6/E7, but is not limited thereto.

The present inventors confirmed through experiments that when the incubating blood cells are subjected to a low temperature treatment, non-enucleated nucleated cells are killed. In an embodiment of the present disclosure, the low temperature treatment step may be performed at a temperature of 0°C or higher and 15°C or lower. Desirably, the low temperature treatment step may be performed at 0°C or higher and 10°C or lower, more desirably 0°C or higher and 7°C or lower, or 0°C or higher and 5°C or lower, and most desirably 3°C or higher and 5°C or lower. The low temperature treatment step may be performed by refrigerating the incubating cells. In an embodiment of the present disclosure, the low temperature treatment step may be performed by refrigeration for 1 to 30 days. The low temperature treatment step may be performed by refrigeration for desirably 3 to 10 days, or 5 to 10 days, and most desirably 7 to 10 days.

According to the present disclosure, when the incubating blood cells are subjected to the low temperature treatment, the death rate of nucleated cells is measured to be 97% or higher. Desirably, during the low temperature treatment, the death rate of nucleated cells is measured to be 98% or higher, more desirably 99% or higher, and most desirably 99.9% or higher. The cell death rate after the low temperature treatment may be measured using any method known in the art. For example, the measurement of the cell death rate may be measured by a flow cytometer, but is not limited thereto.

In an embodiment of the present disclosure, the method may further include a step of passing the incubating blood cells through a leukocyte-reducing filter to kill only nucleated cells in the incubating blood cells. The step is desirably performed after the low temperature treatment step, but is not limited thereto.

In yet another embodiment of the present disclosure, the method may further include a step of irradiating the incubating blood cells to kill only the nucleated cells in the incubating blood cells. The irradiating step may be performed before the low temperature treatment step or may also be performed after the low temperature treatment step. The radiation dose may be used in various intensities. For example, the total radiation dose to be administered may be about 10 Gy (gray) to about 60 Gy, about 10 Gy to about 50 Gy, about 20 Gy to about 50 Gy, about 20 Gy to about 45 Gy, about 20 Gy to about 35 Gy, about 20 Gy to about 30 Gy, or about 25 Gy to about 30 Gy, but is not limited thereto.

In an embodiment of the present disclosure, the method may further include a step of treating the incubating blood cells with nuclease. In the present disclosure, the term "nuclease" is a general term for nucleic acid decomposing enzymes, and may vary depending on a use and a location. In general, the nuclease may cause a reaction that breaks diphosphorylation bonds of a nucleic acid molecule such as DNA or RNA, and examples of the nuclease include endonuclease that cleaves the inside of a nucleic acid and exonuclease that cleaves the end of the nucleic acid. The nuclease additionally treated in the present disclosure may cleave nucleic acids released from the nuclei or cells present in a culture medium, and the like.

According to the present disclosure, the enucleated blood cells collected from the low temperature-treated cells may not be substantially hemolyzed. In an embodiment of the present disclosure, the enucleated blood cells may be enucleated erythrocytes, and the hemolysis rate (%) of the enucleated erythrocytes may be less than 5, desirably less than 3, less than 1, and more desirably less than 0.8.

Hereinafter, preferred Examples and Experimental Examples will be presented in order to assist the understanding of the present disclosure. However, the following Examples and Experimental Examples are just provided to more easily understand the present disclosure, and the contents of the present disclosure are not limited by Examples or Experimental Examples.

[Example]

Experimental method

1) Sample collection

For fresh erythrocyte samples, an experiment was conducted on the residual blood of patients aged 20 to 60 years who had completed tests and whose erythrocyte count was within a normal range (hemoglobin 12.0 g/dL or higher) among specimens for general blood tests after obtaining consent. For collection of umbilical cord blood, the consent was obtained from healthy mothers, and then the umbilical cord blood was collected after birth to isolate CD34+ cells. In addition, K562 erythroleukemia cells or K562 cells overexpressing HPV16 E6/E7, and erythroid progenitor cell lines were used.

2) Cell production and incubation

A lentiviral tetracycline-regulated system (lentiviral Tet-on system) capable of regulating gene expression with tetracycline was used to construct erythroid progenitor cell lines overexpressing HPV16 E6/E7. When inducing erythrocyte differentiation from the erythroid progenitor cell lines, antibiotics were removed and it was shown using PCR that HPV16 E6/E7 mRNA was not expressed.

The K562 cells were incubated in a RPMI 1640 medium supplemented with 10% fetal bovine serum (FBS), and the medium was replaced with a fresh medium supplemented with doxycycline (1 $\mu$g/mL) every 2 to 3 days.

The erythroid progenitor cell line was incubated in a Stemspan SFEM medium supplemented with EPO (3 IU/ml), SCF (50 ng/ml), and dexamethasone (1 $\mu$M). To provide an environment in which the cell line grew well, some conditions were replaced with a fresh medium at a concentration of $2.5 \times 10^5$ cells/ml by adding 10% FBS every 2 to 3 days.

The FBS was used with Tet system-approved FBS without containing a tetracycline-derived substance to exclude a background that may affect the tetracycline-induced system.

3) Irradiation

A gamma irradiation device Biobeam8000 (STS Steuerungstechnik &. Strahlenschutz GmbH, Braunschweig, Germany, Cs-137) was used to irradiate at radiation doses of 0 Gy, 25 Gy, 35 Gy, and 45 Gy (radiation dose of approximately 1.4 Gy/min).

4) Measurement of hemolysis rate of erythrocytes

Blood was placed in a 1.5 ml tube and centrifuged at 2000 Xg for 10 minutes at 10°C, and 100 $\mu$l of the supernatant (plasma layer) was placed in a 96-well plate. After measuring the absorbance at 415 nm, 380 nm, and 450 nm using a spectrophotometer, plasma hemoglobin (Hb) (mg/dL) was calculated using the following Equation.

$$\text{Plasma Hb}\left(\frac{\text{mg}}{\text{dL}}\right) = 83.6 \times (2 \times A415 - A380 - A450)$$

In addition, the hemolysis rate of erythrocytes was calculated according to Equation below. (Hct = hematocrit)

$$\text{Hemolysis rate of erythrocytes (\%)} = \frac{\text{Plasma Hb} \times (100 - \%\text{Hct})}{\text{Total Hb}} \times 100$$

5) Study on apoptosis using flow cytometry

A flow cytometer Canto II (BD Biosciences, San Jose, CA) was used to measure apoptosis after irradiation and 4°C low temperature treatment. The cells were harvested, washed with phosphate-buffered saline (PBS), suspended in a 1X binding buffer, and the sample was added with each 5 $\mu$l of annexin V and propidium iodide (PI; BD Biosciences) and reacted for 15 minutes at room temperature under light blocking, and then the apoptosis was measured using a flow cytometer.

6) Measurement of DNA fragmentation

The cells were harvested and washed with PBS, and then genomic DNA was extracted using the PureLink genomic DNA mini kit (Invitrogen). 200 ng of purified genomic DNA was loaded on a 1% agarose gel and electrophoresis was performed at 50 V of voltage for 75 minutes.

7) PCR

Pre-denaturation was performed using Amfisure PCR master mix (GenDEPOT) at 96°C for 30 seconds, and then 35 cycles of denaturation at 96°C for 15 seconds, annealing at 65°C for 30 seconds, and extension at 72°C for 20 seconds were performed. The primers used were as follows.

[Table 1]

| HPV16 E6/E7 | |
|---|---|
| Forward (SEQ ID NO: 1) | 5'-GTCAAAAGCCACTGTGTCCTG-3' |
| Reverse (SEQ ID NO: 2) | 5'-GATTTGCAACCAGAGACAACTG-3' |

[Table 2]

| GAPDH | |
|---|---|
| Forward (SEQ ID NO: 3) | 5'-GAAGGTGAAGGTCGGAGTC-3' |
| Reverse (SEQ ID NO: 4) | 5'-GACAAGCTTCCCGTTCTCAG-3' |

Experimental Example 1. Confirmation of expression of overexpressed HPV16 E6/E7 after removal of doxycycline

To confirm whether the expression of oncogene HPV16 E6/E7 was well regulated by a Tet-on system regulated by the presence or absence of doxycycline, an experiment was conducted to remove doxycycline from K562 cells overexpressing HPV16 E6/E7. It was confirmed by cell fluorescence imaging and PCR technique that gene expression was not completely turned off due to a leakage effect, in which the background of the vector was expressed even in the absence of doxycycline.

An Enhanced Green Fluorescent Protein (eGFP) sequence was designed to be expressed upstream of HPV16 E6/E7, and when the doxycycline was present, a transactivator bound to a TRE promoter to regulate the expression.

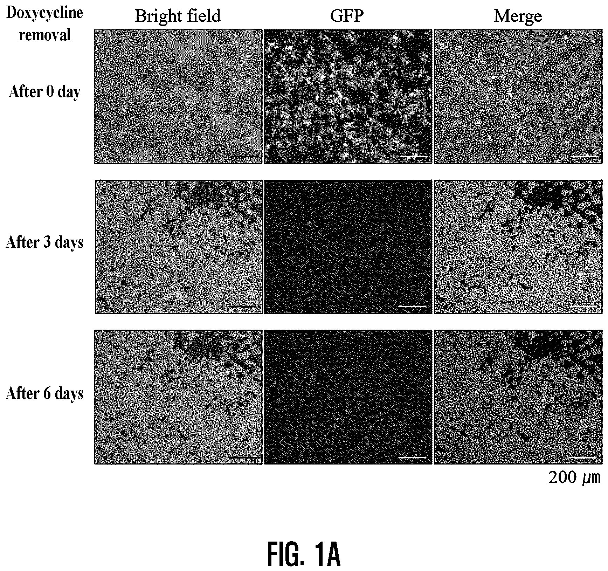

The cells were washed twice with PBS, and then the medium was replaced with a doxycycline-free medium, and the fluorescence expression levels after 3 and 6 days were compared with those when the cells were grown in the doxycycline-free medium. It was observed under a fluorescence microscope that eGFP fluorescence was not completely eliminated (FIG. 1A). FIG. 1A is a cell fluorescence photograph (Scale bar = 200 $\mu$m) taken at 0, 3, and 6 days after doxycycline was removed from a K562 cell culture overexpressing eGFP-HPV16 E6/E7.

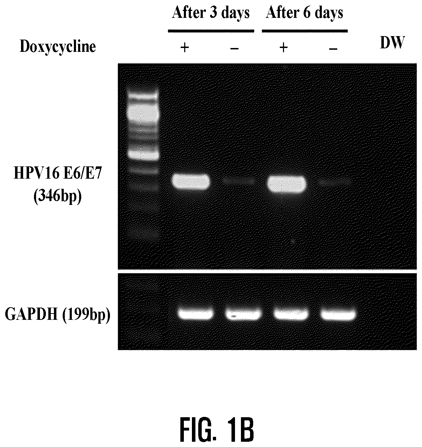

To confirm the mRNA level of HPV16 E6/E7 after doxycycline removal, the cells were harvested at 3 and 6 days after doxycycline removal, RNA was extracted, and cDNA was synthesized. After PCR gene amplification, the presence of HPV16 E6/E7 mRNA was shown using electrophoresis (FIG. 1B). FIG. 1B shows the mRNA expression of HPV16 E6/E7 confirmed by post-PCR electrophoresis after 3 and 6 days of incubation depending on the presence or absence of doxycycline, and Gylceraldehyde 3-phosphate dehydrogenase (GAPDH) was used as an internal control.

As a result, although the tetracycline-induced expression system selectively suppressed oncogene expression, as a result of observation by fluorescence microscopy and post-PCR electrophoresis, it was confirmed that the Tet-on system, in which the HPV16 E6/E7 gene was expressed even after doxycycline was removed, was not perfect, and there was a problem with the continued expression of introduced genes (background expression). That is, although erythrocytes as the final product have no nucleus due to the enucleation process, it may be confirmed that a method for killing nucleated cells remaining in the final erythrocyte product is required to secure stability suitable for clinical application.

Experimental Example 2. Test of hemolyzed erythrocyte ratio after irradiation

To determine the optimal radiation absorbed dose that may damage nucleated cells while minimizing hemolysis of erythrocytes, the hemolysis rate of erythrocytes at 0 day after irradiation and 7 days after refrigeration (4°C) was calculated using the following Equation.

$$\text{Hemolysis (\%)} = \frac{\text{Plasma Hb} \times (100 - \%\text{Hct})}{\text{Total Hb}}$$

$$\text{- Plasma Hb (mg/dl)} = 83.6 \times (2 \times A_{415} - A_{380} - A_{450})$$

- Hb: Plasma hemoglobin (Hemoglobin)
- A415: Absorbance at 415 nm wavelength
- A380: Absorbance at 380 nm wavelength
- A450: Absorbance at 450 nm wavelength
- Hct: Hemolysis rate of erythrocytes (Hematocrit, ratio of erythrocytes in blood)

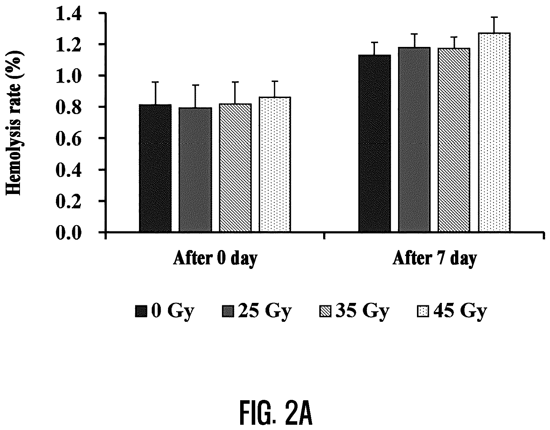

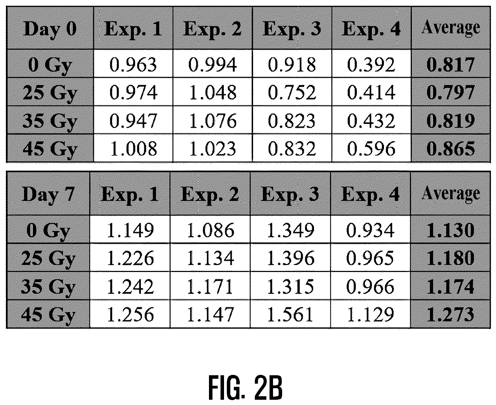

The hemolysis rates of erythrocytes were compared at 25 Gy, 35 Gy, and 45 Gy relative to 0 Gy of radiation dose after 7 days of irradiation, and there was no significant difference in hemolysis rate after 7 days of refrigeration according to a radiation dose (n = 4, 0 Gy vs 25 Gy: p = 0.699, 0 Gy vs 35 Gy: p = 0.714, 0 Gy vs 45 Gy: p = 0.318 Exp.: experiment). The hemolysis rates of erythrocytes immediately after irradiation were average 0.817, 0.797, 0.819, and 0.865 for 0 Gy, 25 Gy, 35 Gy, and 45 Gy, respectively, and after 7 days of refrigeration (4°C), the hemolysis rates were 1.130, 1.180, 1.174, and 1.273, respectively (FIGS. 2A and 2B).

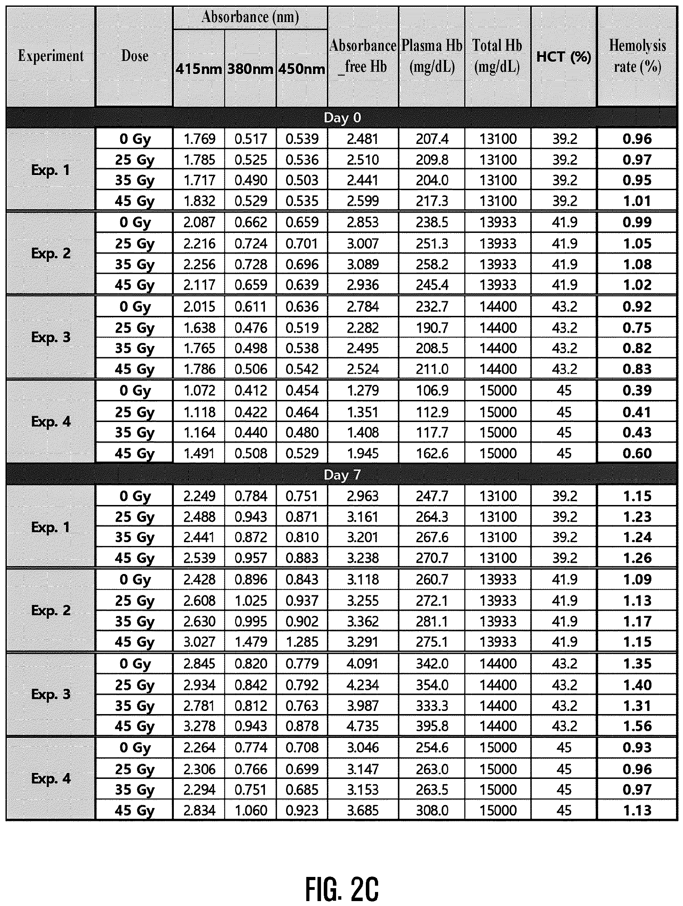

The absorbance measured to calculate the hemolysis rate of erythrocytes and the hemolysis rate of erythrocytes after irradiation were shown in FIG. 2C. The evaluation criteria for the hemolysis rate of erythrocytes are less than 1% based on the U.S. Food and Drug Administration (FDA) and less than 0.8% based on the European Council of Europe (CE).

Experimental Example 3. Study on apoptosis according to radiation dose of nucleated cells

To ensure stability suitable for clinical application, it is necessary to remove nucleated cells remaining in the final erythrocyte product, and as an experiment for verifying the removal, K562 cells overexpressing oncogene HPV16 E6/E7 were irradiated at 0, 25, 35, and 45 Gy of radiation.

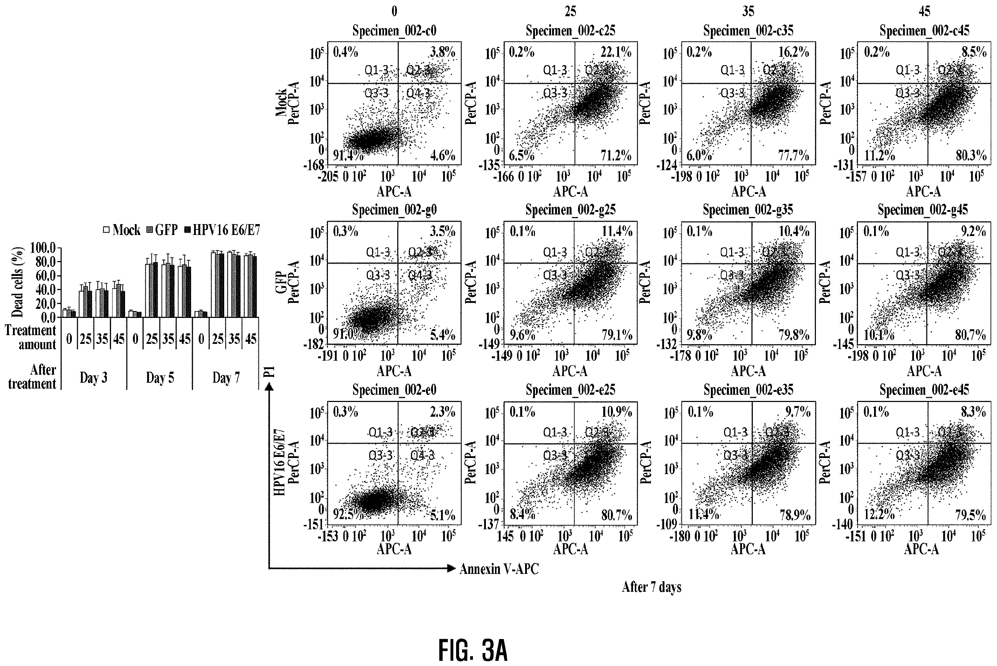

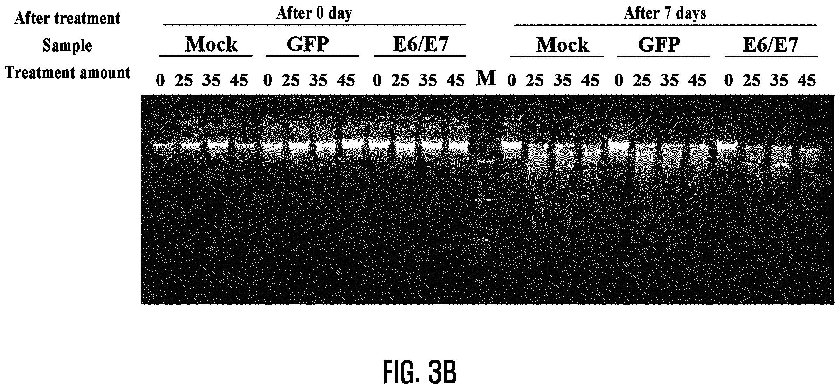

After irradiation, cells were incubated at 37°C, and flow cytometry was performed using Annexin V/PI staining after 3, 5, and 7 days of irradiation to measure apoptosis. As a result, 7 days after irradiation, a mock (a control group in which only a reagent was added without transduction), an eGFP control group, and a HPV16 E6/E7 overexpression sample all showed approximately 90% of apoptosis, and there was no significant difference between the samples (FIG. 3A). FIG. 3A shows results of flow cytometry after annexin v/PI staining at 3, 5, and 7 days after irradiation of 0, 25, 35, and 45 Gy of radiation to mock cells without virus treatment, eGFP control, or K562 cells overexpressing HPV16 E6/E7. In addition, after extracting genomic DNA from the sample, the fragmented DNA was confirmed through electrophoresis (FIG. 3B). FIG. 3B shows results of electrophoresis performed at 50 V of voltage for 75 minutes by loading 200 ng of purified genomic DNA on a 1% agarose gel after extracting the genomic DNA from the sample of FIG. 3A.

Even by increasing the radiation dose from 25 Gy to 35 and 45 Gy, there was no significant increase in the apoptosis rate. As a result, it may be confirmed that complete apoptosis of nucleated cells is not induced only by irradiation. In addition, the radiation dose widely used for blood products in domestic medical institutions was 25 Gy (The Korean Journal of Blood Transfusion, Vol. 27, No. 2, Current Status of Irradiated Blood Components and Blood Irradiators in Korean Medical Institutions, 2016), and thus the radiation dose was set to 25 Gy in subsequent experiments.

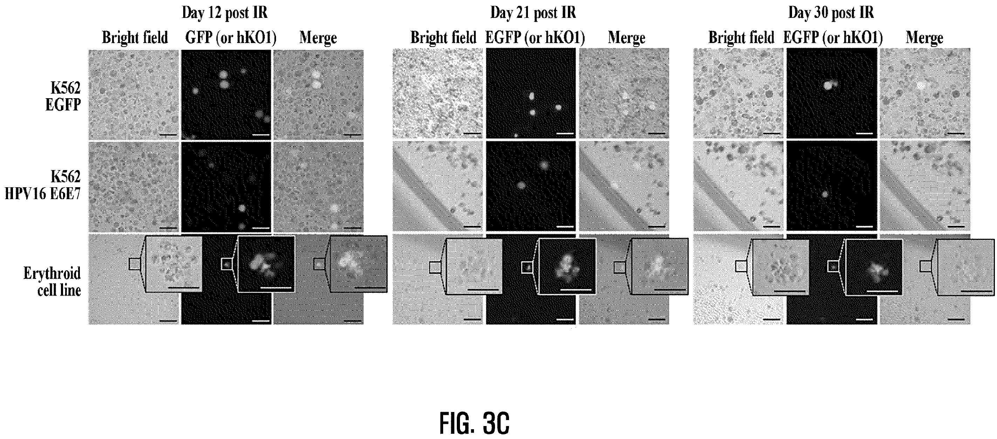

K562 cells overexpressing GFP or HPV16 E6/E7 and an erythroid progenitor cell line overexpressing HPV16 E6/E7 were irradiated with 25 Gy of radiation, and then incubated at 37°C and the expression of fluorescence was observed, and cells expressing fluorescence could be observed up to 30 days in incubation (FIG. 3C). It is shown that irradiation alone is not sufficient for nucleated cell death, as safety issues may occur when applied clinically.

Experimental Example 4. Study on apoptosis according to low temperature treatment (4°C) of nucleated cells

As confirmed in Experimental Example 3, since irradiation did not induce complete apoptosis, to confirm whether complete apoptosis may be induced by refrigeration at 4°C, the degree of apoptosis was compared using trypan blue staining after refrigeration at 4°C in K562 cells overexpressing HPV16 E6/E7 and an erythroid progenitor cell line.

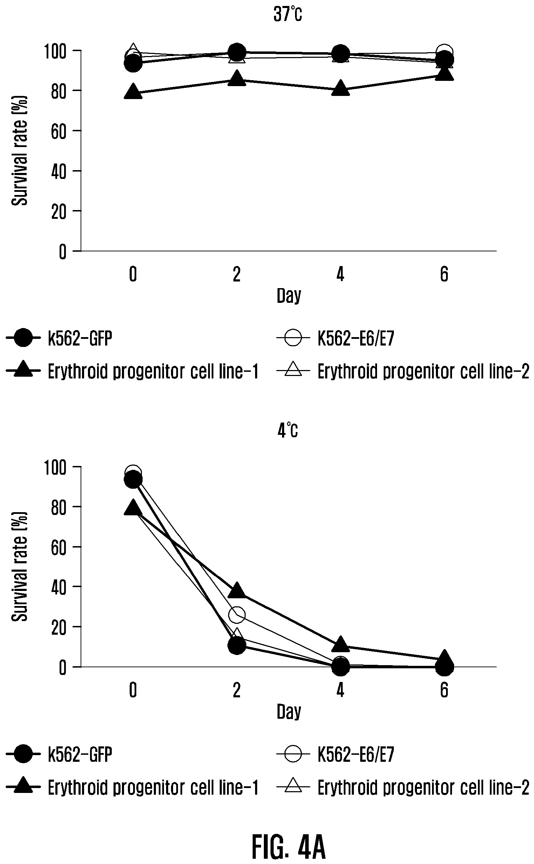

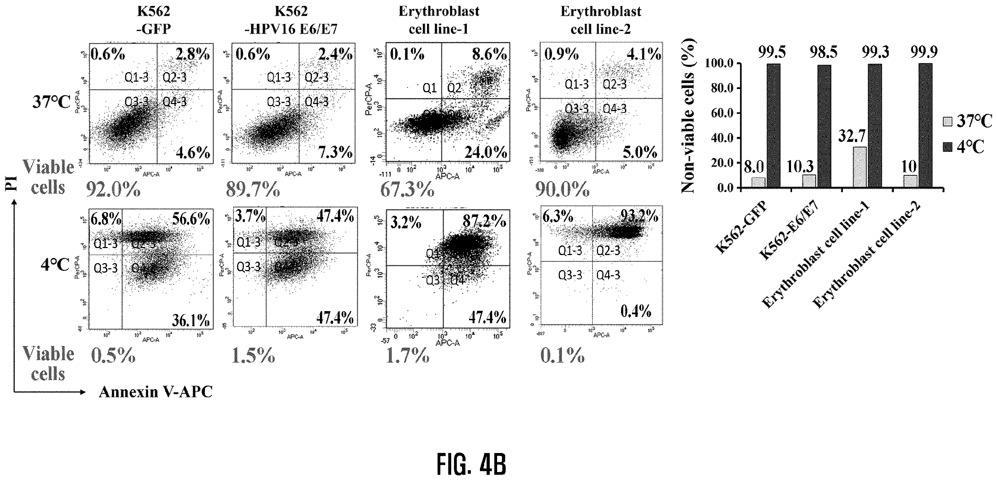

During incubation at 37°C, a cell survival rate of 75% or higher was shown, but during refrigeration at 4°C, the cell survival rate was decreased immediately, and on day 6 of storage, K562 and erythroid progenitor cell line-2 showed the cell survival rate of less than 1%, and erythroid progenitor cell line-1 showed the cell survival rate of about 3% (trypan blue staining). After day 6 of refrigeration at 4°C, as a result of measuring the apoptosis rate by flow cytometry using annexin V/PI staining, an apoptosis rate of 98.5% or higher was shown (FIGS. 4A and 4B). FIG. 4A illustrates a result of measuring the cell survival rate when nucleated cells were stored in a refrigerator at 4°C, in which when the K562 cells, the K562 cells overexpressing HPV16 E6/E7 and the erythroid progenitor cell lines-1 and -2 were incubated at 37°C and stored in a refrigerator at 4°C, the cell survival rates were measured by staining with trypan blue. FIG. 4B illustrates results of measuring the cell survival rate through flow cytometry after staining cells with Annexin V/PI staining at 6 days after refrigeration at 4°C.

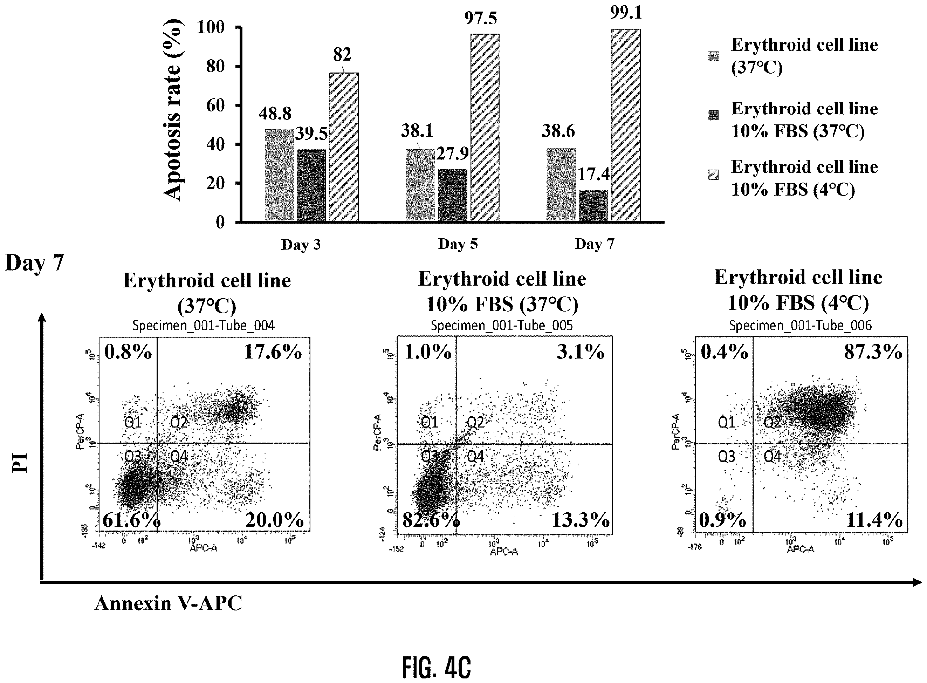

When maintaining and incubating the erythroid progenitor cell lines, serum was not added, and then the medium was added with the serum when differentiating into erythrocytes, and thus apoptosis was measured by flow cytometry after annexin V/PI staining after 3, 5, and 7 days according to the conditions. The refrigeration was performed by adding 10% fetal bovine serum (FBS) to the medium, similarly to the storage conditions for erythrocytes (FIG. 4C). FIG. 4C illustrates results of measuring the cell survival rates of the erythroid progenitor cell line overexpressing HPV16 E6/E7 after 7 days of incubation at 37°C (serum +/-) and refrigeration at 4°C (serum +).

As a result, the apoptosis was observed as 82% after 3 days, 97.5% after 5 days, and 99.1% after 7 days of 4°C refrigeration.

Experimental Example 5. Comparison of apoptosis according to irradiation and low temperature treatment

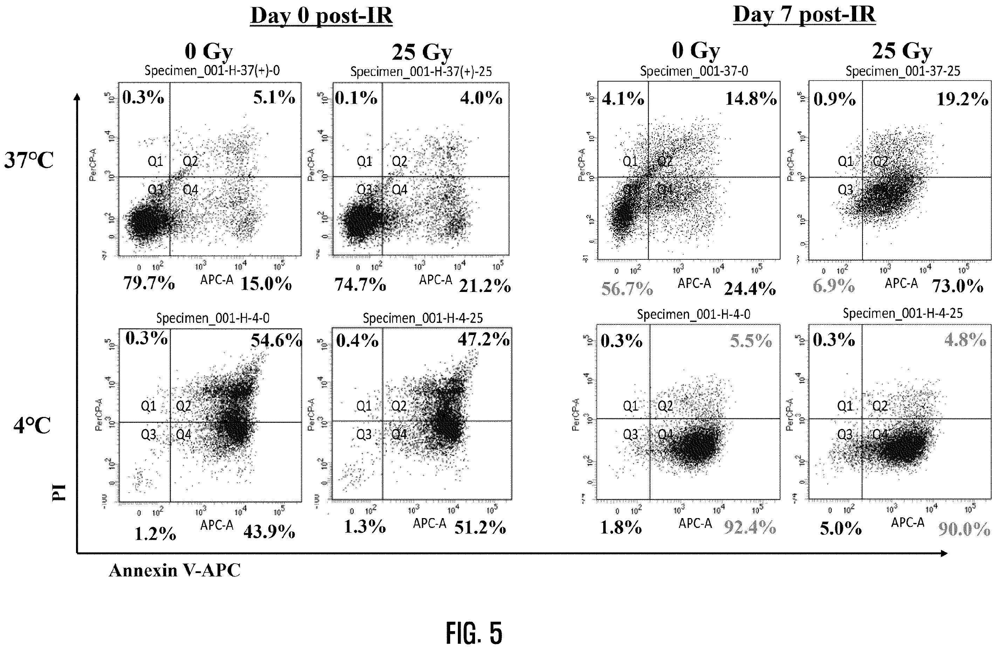

In order to confirm induction of apoptosis by a combination of irradiation (25 Gy) and refrigeration (4°C) in addition to irradiation (25 Gy) or refrigeration (4°C) alone, after culturing and storing for 4 days at 37°C and 4°C in a medium supplemented with 10% serum, cells were treated with irradiation (25 Gy) and Annexin V/PI staining was performed after 4 hours (IR-Day 0) and 7 days (IR-Day 7), and apoptosis was measured using flow cytometry. As a result, the apoptosis rate (98.2%) in a non-irradiated condition was higher than the apoptosis rate (95.1%) in the case of combined treatment with irradiation (25 Gy) and refrigeration (FIG. 5).

Experimental Example 6. Experiment on removal of erythroid progenitor cells using leukocyte-reducing filter

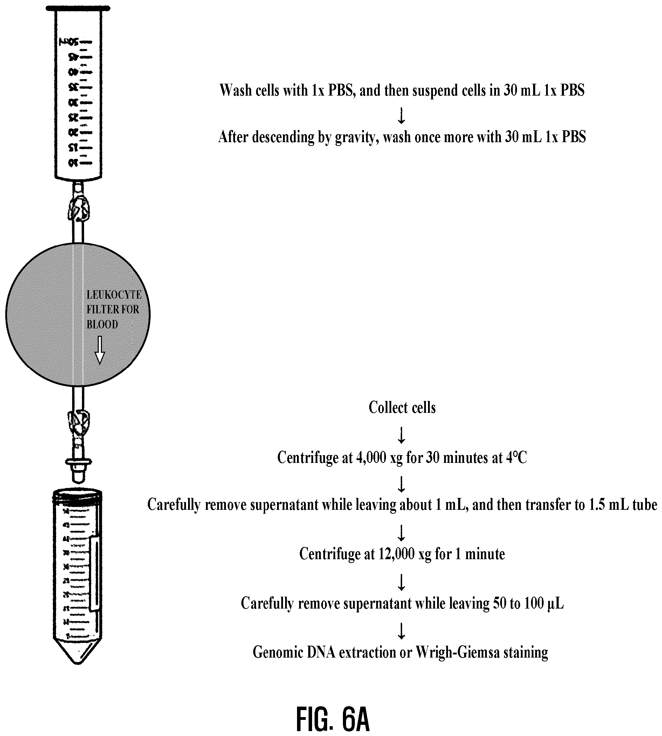

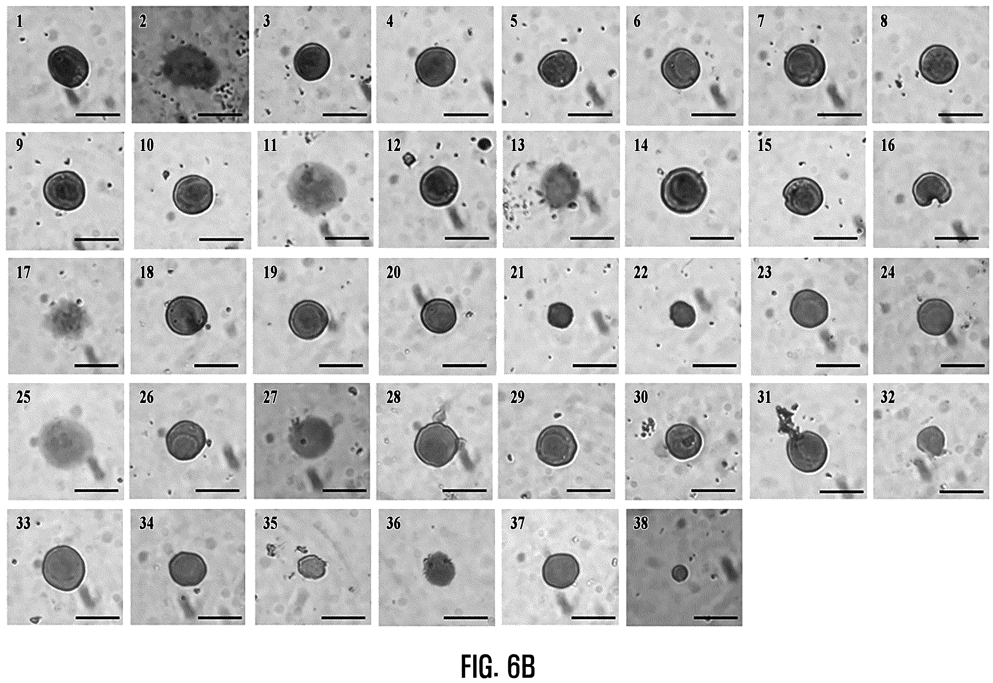

To determine how much nucleated cells may be removed using a leukocyte-reducing filter, $5.58 \times 10^7$ cells of erythroid progenitor cells passed through a BioR 02 plus BS PF (Fresenius kabi, A2BB0080) leukocyte-reducing filter and contained in a 50-ml conical tube (FIG. 6A). After passing through the leukocyte-reducing filter and centrifuging for 20 minutes, the collected cells were stained with Wright-Giemsa, and a total of 38 cells were observed (Scale bar = 20 $\mu$m) (FIG. 6B).

Since 99.9% or more of cells may be removed, but some passing cells are present by using the leukocyte-reducing filter, nucleated cells may not be completely removed only by the leukocyte-reducing filter, and the removal rate of erythroid progenitor cells may be increased by using a method of using the leukocyte-reducing filter in a state where most cells die by refrigeration at 4°C.

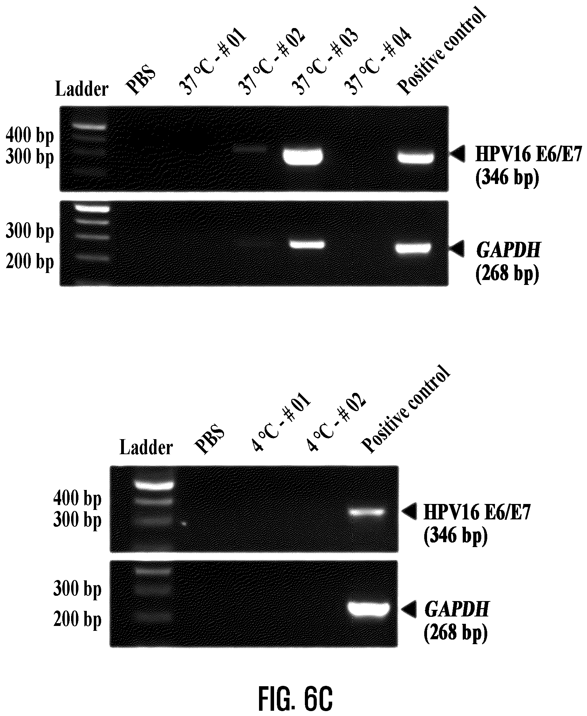

The erythroid progenitor cell lines incubated at 37°C and 4°C for 7 days each passed through the leukocyte-reducing filter, and then genomic DNA was extracted, amplified by PCR, and confirmed by electrophoresis to determine whether there were any nucleated cells remaining in a filtrate. As a result of the experiment repeated four times under the condition of 37°C and repeated twice under the condition of 4°C, it was verified that there were no nucleated cells because genomic DNA was not amplified when passing through the leukocyte-reducing filter after incubation at 4°C (FIG. 6C).

**Claims**

1.   A nucleated cell death induction method in enucleated blood cells, comprising:

   incubating hematopoietic stem cells or progenitor cells into enucleated blood cells; and
   killing nucleated cells by a low temperature treatment of the incubating blood cells.

2.   The nucleated cell death induction method of claim 1, wherein the hematopoietic stem cells or progenitor cells are isolated from bone marrow, umbilical cord blood, or peripheral blood, or derived from adult stem cells, erythroid progenitor cell lines, platelet progenitor cell lines, embryonic stem cells or iPS cells.

3.   The nucleated cell death induction method of claim 1, wherein the

   progenitor cells comprise erythroid progenitor cells, and
   the erythroid progenitor cells are selected from the group consisting of burst forming unit erythroids (BFU-E), colony forming unit erythroids (CFU-E), proerythroblasts, basophilic erythroblasts, polychromatic erythroblasts, and orthochromatic erythroblasts.

4.   The nucleated cell death induction method of claim 1, wherein the enucleated blood cells comprise enucleated erythrocytes or enucleated platelet cells.

5.   The nucleated cell death induction method of claim 4, wherein the enucleated erythrocytes are selected from the group consisting of reticulocytes and erythrocytes.

6.   The nucleated cell death induction method of claim 1, wherein the nucleated cells are erythroid progenitor cells or platelet progenitor cells that overexpress proliferation-related genes.

7.   The nucleated cell death induction method of claim 6, wherein the proliferation-related gene is HPV16 E6/E7.

8.   The nucleated cell death induction method of claim 1, wherein the low temperature treatment is performed by refrigeration for 3 to 10 days.

9. The nucleated cell death induction method of claim 1, wherein the low temperature treatment is performed at 0°C or higher and 15°C or lower.

10. The nucleated cell death induction method of claim 1, wherein the low temperature treatment is performed at 0°C or higher and 5°C or lower.

11. The nucleated cell death induction method of claim 1, further comprising: passing the incubating blood cells through a leukocyte-reducing filter.

12. The nucleated cell death induction method of claim 1, further comprising: irradiating the incubating blood cells.

13. The nucleated cell death induction method of claim 1, further comprising: treating nuclease.

14. The nucleated cell death induction method of claim 1, wherein the death rate of nucleated cells in the low temperature treated cells is 97% or higher.

200 μm

FIG. 1A

FIG. 1B

FIG. 2A

| Day 0 | Exp. 1 | Exp. 2 | Exp. 3 | Exp. 4 | Average |
|-------|--------|--------|--------|--------|---------|
| 0 Gy  | 0.963  | 0.994  | 0.918  | 0.392  | **0.817** |
| 25 Gy | 0.974  | 1.048  | 0.752  | 0.414  | **0.797** |
| 35 Gy | 0.947  | 1.076  | 0.823  | 0.432  | **0.819** |
| 45 Gy | 1.008  | 1.023  | 0.832  | 0.596  | **0.865** |

| Day 7 | Exp. 1 | Exp. 2 | Exp. 3 | Exp. 4 | Average |
|-------|--------|--------|--------|--------|---------|
| 0 Gy  | 1.149  | 1.086  | 1.349  | 0.934  | **1.130** |
| 25 Gy | 1.226  | 1.134  | 1.396  | 0.965  | **1.180** |
| 35 Gy | 1.242  | 1.171  | 1.315  | 0.966  | **1.174** |
| 45 Gy | 1.256  | 1.147  | 1.561  | 1.129  | **1.273** |

# FIG. 2B

| Experiment | Dose | Absorbance (nm) | | | Absorbance _free Hb | Plasma Hb (mg/dL) | Total Hb (mg/dL) | HCT (%) | Hemolysis rate (%) |
|---|---|---|---|---|---|---|---|---|---|
| | | 415nm | 380nm | 450nm | | | | | |
| Day 0 | | | | | | | | | |
| Exp. 1 | 0 Gy | 1.769 | 0.517 | 0.539 | 2.481 | 207.4 | 13100 | 39.2 | 0.96 |
| | 25 Gy | 1.785 | 0.525 | 0.536 | 2.510 | 209.8 | 13100 | 39.2 | 0.97 |
| | 35 Gy | 1.717 | 0.490 | 0.503 | 2.441 | 204.0 | 13100 | 39.2 | 0.95 |
| | 45 Gy | 1.832 | 0.529 | 0.535 | 2.599 | 217.3 | 13100 | 39.2 | 1.01 |
| Exp. 2 | 0 Gy | 2.087 | 0.662 | 0.659 | 2.853 | 238.5 | 13933 | 41.9 | 0.99 |
| | 25 Gy | 2.216 | 0.724 | 0.701 | 3.007 | 251.3 | 13933 | 41.9 | 1.05 |
| | 35 Gy | 2.256 | 0.728 | 0.696 | 3.089 | 258.2 | 13933 | 41.9 | 1.08 |
| | 45 Gy | 2.117 | 0.659 | 0.639 | 2.936 | 245.4 | 13933 | 41.9 | 1.02 |
| Exp. 3 | 0 Gy | 2.015 | 0.611 | 0.636 | 2.784 | 232.7 | 14400 | 43.2 | 0.92 |
| | 25 Gy | 1.638 | 0.476 | 0.519 | 2.282 | 190.7 | 14400 | 43.2 | 0.75 |
| | 35 Gy | 1.765 | 0.498 | 0.538 | 2.495 | 208.5 | 14400 | 43.2 | 0.82 |
| | 45 Gy | 1.786 | 0.506 | 0.542 | 2.524 | 211.0 | 14400 | 43.2 | 0.83 |
| Exp. 4 | 0 Gy | 1.072 | 0.412 | 0.454 | 1.279 | 106.9 | 15000 | 45 | 0.39 |
| | 25 Gy | 1.118 | 0.422 | 0.464 | 1.351 | 112.9 | 15000 | 45 | 0.41 |
| | 35 Gy | 1.164 | 0.440 | 0.480 | 1.408 | 117.7 | 15000 | 45 | 0.43 |
| | 45 Gy | 1.491 | 0.508 | 0.529 | 1.945 | 162.6 | 15000 | 45 | 0.60 |
| Day 7 | | | | | | | | | |
| Exp. 1 | 0 Gy | 2.249 | 0.784 | 0.751 | 2.963 | 247.7 | 13100 | 39.2 | 1.15 |
| | 25 Gy | 2.488 | 0.943 | 0.871 | 3.161 | 264.3 | 13100 | 39.2 | 1.23 |
| | 35 Gy | 2.441 | 0.872 | 0.810 | 3.201 | 267.6 | 13100 | 39.2 | 1.24 |
| | 45 Gy | 2.539 | 0.957 | 0.883 | 3.238 | 270.7 | 13100 | 39.2 | 1.26 |
| Exp. 2 | 0 Gy | 2.428 | 0.896 | 0.843 | 3.118 | 260.7 | 13933 | 41.9 | 1.09 |
| | 25 Gy | 2.608 | 1.025 | 0.937 | 3.255 | 272.1 | 13933 | 41.9 | 1.13 |
| | 35 Gy | 2.630 | 0.995 | 0.902 | 3.362 | 281.1 | 13933 | 41.9 | 1.17 |
| | 45 Gy | 3.027 | 1.479 | 1.285 | 3.291 | 275.1 | 13933 | 41.9 | 1.15 |
| Exp. 3 | 0 Gy | 2.845 | 0.820 | 0.779 | 4.091 | 342.0 | 14400 | 43.2 | 1.35 |
| | 25 Gy | 2.934 | 0.842 | 0.792 | 4.234 | 354.0 | 14400 | 43.2 | 1.40 |
| | 35 Gy | 2.781 | 0.812 | 0.763 | 3.987 | 333.3 | 14400 | 43.2 | 1.31 |
| | 45 Gy | 3.278 | 0.943 | 0.878 | 4.735 | 395.8 | 14400 | 43.2 | 1.56 |
| Exp. 4 | 0 Gy | 2.264 | 0.774 | 0.708 | 3.046 | 254.6 | 15000 | 45 | 0.93 |
| | 25 Gy | 2.306 | 0.766 | 0.699 | 3.147 | 263.0 | 15000 | 45 | 0.96 |
| | 35 Gy | 2.294 | 0.751 | 0.685 | 3.153 | 263.5 | 15000 | 45 | 0.97 |
| | 45 Gy | 2.834 | 1.060 | 0.923 | 3.685 | 308.0 | 15000 | 45 | 1.13 |

# FIG. 2C

FIG. 3A

FIG. 3B

FIG. 3C

**37℃**

Survival rate [%]

Day

●— k562-GFP　　　　　　　─○─ K562-E6/E7

▲— Erythroid progenitor cell line-1　─△─ Erythroid progenitor cell line-2

**4℃**

Survival rate [%]

Day

●— k562-GFP　　　　　　　─○─ K562-E6/E7

▲— Erythroid progenitor cell line-1　─△─ Erythroid progenitor cell line-2

# FIG. 4A

**FIG. 4B**

FIG. 4C

**Day 0 post-IR**        **Day 7 post-IR**

FIG. 5

Wash cells with 1x PBS, and then suspend cells in 30 mL 1x PBS
↓
After descending by gravity, wash once more with 30 mL 1x PBS

LEUKOCYTE
FILTER FOR
BLOOD
⇩

Collect cells
↓
Centrifuge at 4,000 xg for 30 minutes at 4°C
↓
Carefully remove supernatant while leaving about 1 mL, and then transfer to 1.5 mL tube
↓
Centrifuge at 12,000 xg for 1 minute
↓
Carefully remove supernatant while leaving 50 to 100 μL
↓
Genomic DNA extraction or Wrigh-Giemsa staining

# FIG. 6A

FIG. 6B

FIG. 6C

## INTERNATIONAL SEARCH REPORT

| International application No. |
|---|
| **PCT/KR2023/004918** |

### A. CLASSIFICATION OF SUBJECT MATTER

**C12N 5/00**(2006.01)i; **C12N 5/078**(2010.01)i

According to International Patent Classification (IPC) or to both national classification and IPC

### B. FIELDS SEARCHED

Minimum documentation searched (classification system followed by classification symbols)

C12N 5/00(2006.01); C12N 5/071(2010.01); C12N 5/078(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 조혈모 세포(hematopoietic stem cell), 적혈구(erythrocyte), 저온 처리(low temperature treatment), 망상 적혈구(reticulocyte), 유핵 세포(karyota)

### C. DOCUMENTS CONSIDERED TO BE RELEVANT

| Category* | Citation of document, with indication, where appropriate, of the relevant passages | Relevant to claim No. |
|---|---|---|
| Y | KR 10-2013-0015481 A (IUCF-HYU (INDUSTRY-UNIVERSITY COOPERATION FOUNDATION HANYANG UNIVERSITY)) 14 February 2013 (2013-02-14)<br>See claims 1, 2 and 8; and paragraphs [0028], [0042], [0045], [0046], [0049], [0083], [0144] and [0147]. | 1-12,14 |
| A | | 13 |
| Y | CHEM, S.-Z. et al. UV irradiation/cold shock-mediated apoptosis is switched to bubbling cell death at low temperatures. Oncotarget. 10 February 2015, vol. 6, no. 10, pp. 8007-8018.<br>See abstract; pages 8009 and 8016; and figures 1B and 2. | 1-12,14 |
| A | KR 10-2011-0094470 A (COLLEGE OF MEDICINE POCHON CHA UNIVERSITY INDUSTRY-ACADEMIC COOPERATION FOUNDATION) 24 August 2011 (2011-08-24)<br>See claims 1 and 3. | 1-14 |
| A | 권오헌 등. 방사선조사가 백혈구제거 적혈구농축액에 미치는 영향. 대한혈액학회지. 1993, vol. 28, no. 1, pp. 135-141 (KWON, Oh-Heon et al. In Vitro Storage Lesions of Filtered RBC after Irradiation. Korean Journal of Hematology.)<br>See page 139. | 1-14 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **19 July 2023** | **19 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2023/004918** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2010-0081678 A (INDUSTRY-ACADEMIC COOPERATION FOUNDATION, YONSEI UNIVERSITY) 15 July 2010 (2010-07-15)<br>See claims 1, 3 and 4. | 1-14 |

Form PCT/ISA/210 (second sheet) (July 2022)

**INTERNATIONAL SEARCH REPORT**

International application No.

**PCT/KR2023/004918**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2023/004918**

| Patent document cited in search report | | Publication date (day/month/year) | Patent family member(s) | | Publication date (day/month/year) |
|---|---|---|---|---|---|
| KR 10-2013-0015481 | A | 14 February 2013 | None | | |
| KR 10-2011-0094470 | A | 24 August 2011 | KR 10-1184919 | B1 | 28 September 2012 |
| KR 10-2010-0081678 | A | 15 July 2010 | KR 10-1178424 | B1 | 30 August 2012 |

Form PCT/ISA/210 (patent family annex) (July 2022)